(19) Europäisches Patentamt / European Patent Office / Office européen des brevets

(11) **EP 2 515 755 B1**

(12) **EUROPEAN PATENT SPECIFICATION**

(45) Date of publication and mention
of the grant of the patent:
**20.04.2016 Bulletin 2016/16**

(21) Application number: **10838409.0**

(22) Date of filing: **20.12.2010**

(51) Int Cl.:
**A61B 5/053** [(2006.01)]

(86) International application number:
**PCT/AU2010/001713**

(87) International publication number:
**WO 2011/075769 (30.06.2011 Gazette 2011/26)**

(54) **ANALYSING IMPEDANCE MEASUREMENTS**

ANALYSE VON IMPEDANZMESSUNGEN

ANALYSE DES MESURES D'IMPÉDANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2009 AU 2009906198**

(43) Date of publication of application:
**31.10.2012 Bulletin 2012/44**

(73) Proprietor: **Impedimed Limited**
**Pinkenba, QLD 4008 (AU)**

(72) Inventors:
• **GAW, Richelle Leanne**
**Coorparoo, Queensland 4151 (AU)**
• **ZIEGELAAR, Brian William**
**Carina Heights, Queensland 4150 (AU)**

(74) Representative: **Appleyard Lees IP LLP**
**15 Clare Road**
**Halifax HX1 2HY (GB)**

(56) References cited:
EP-A1- 1 114 610      EP-A1- 1 338 246
EP-A1- 1 452 131      WO-A1-2007/041783
WO-A1-2009/100491      JP-A- 2001 321 352

JP-A- 2008 022 995      SU-A1- 1 132 911
US-A- 4 144 878      US-A- 4 314 563
US-A1- 2005 177 062      US-A1- 2009 264 776
US-A1- 2009 287 102

• GOLDEN J C ET AL: "Assessment of peripheral hemodynamics using impedance plethysmography.", PHYSICAL THERAPY OCT 1986, vol. 66, no. 10, October 1986 (1986-10), pages 1544-1547, XP002695077, ISSN: 0031-9023
• KIM Y ET AL: "Impedance tomography and its application in deep venous thrombosis detection", IEEE ENGINEERING IN MEDICINE AND BIOLOGY MAGAZINE, IEEE SERVICE CENTER, PISACATAWAY, NJ, US, vol. 8, no. 1, 1 March 1989 (1989-03-01), pages 46-49, XP011437120, ISSN: 0739-5175, DOI: 10.1109/51.32405
• FENECH M ET AL: "Extracellular and Intracellular Volume Variations During Postural Change Measured by Segmental and Wrist-Ankle Bioimpedance Spectroscopy", IEEE TRANSACTIONS ON BIOMEDICAL ENGINEERING, IEEE SERVICE CENTER, PISCATAWAY, NJ, USA, vol. 51, no. 1, 1 January 2004 (2004-01-01), pages 166-175, XP011104178, ISSN: 0018-9294, DOI: 10.1109/TBME.2003.820338
• SCHARFETTER H ET AL: "Effect of postural changes on the reliability of volume estimations from bioimpedance spectroscopy data.", KIDNEY INTERNATIONAL APR 1997, vol. 51, no. 4, April 1997 (1997-04), pages 1078-1087, ISSN: 0085-2538

**Description**

**Background of the Invention**

The present invention relates to a method and apparatus for use in analysing impedance measurements performed on a subject, and in particular, to a method and apparatus for determining an indicator indicative of changes in body fluid levels in the subject over time, the indicator being used in the assessment of venous insufficiency.

**Description of the Prior Art**

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

Venous insufficiency is a condition characterized by an inability for veins to adequately return blood to the heart. Normally, when a subject is in a standing position, the blood in the subject's leg veins is urged back towards the heart against gravity by a combination of mechanisms, such as muscular squeezing of the leg veins, and through the action of one-way valves in the veins. However, conditions can arise such as increased pressure within the veins, deep vein thrombosis (DVT), phlebitis, or the like, which lead to blood pooling in the legs.

Chronic venous disease (CVD) is common with a 3-7% prevalence, resulting in an economic cost US$1 billion per annum.

Typical detection methods for venous insufficiency involve examining for physical symptoms such as swelling in the leg or ankle, tightness in the calves, leg tiredness, pain while walking, or the like. Venous insufficiency may also be associated with varicose veins.

Other techniques for assessing venous insufficiency include measuring the ambulatory venous pressure, which is achieved by inserting a needle into the vein on the dorsum of the foot. Whilst this is regarded as the gold standard of haemodynamic investigation, this is invasive, and it is therefore desirable to find alternative non-invasive techniques. Two such methods are air plethysmography (APG) and strain gauge plethysmography (SPG).

SPG involves placing mercury strain gauges in a silastic band around the calf muscle which are calibrated to read percentage leg volume changes, as described for example in Nicolaides AN (2000) "Investigation of Chronic Venous Insufficiency: A Consensus Statement" Circulation 102:126-163. These measurements are typically performed during exercise regimens to allow venous refilling time and the ejection volume to be assessed. APG uses an air bladder which surrounds the leg from the knee to the ankle. The bladder is inflated to a known pressure, with volume changes in the calf muscle being determined based on changes in pressure on the bladder during a sequence of postural changes.

However, these techniques are only of limited accuracy, and can require extensive calibration and exercise, to allow useable measurement to be obtained.

Lymphoedema is a condition characterised by excess protein and oedema in the tissues as a result of reduced lymphatic transport capacity and/or reduced tissue proteolytic capacity in the presence of a normal lymphatic load. Acquired, or secondary lymphoedema, is caused by damaged or blocked lymphatic vessels. The commonest inciting events are surgery and/or radiotherapy. However, onset of lymphoedema is unpredictable and may develop within days of its cause or at any time during a period of many years after that cause.

One existing technique for determining biological parameters relating to a subject, such as fluid levels, involves the use of bioelectrical impedance. This involves measuring the electrical impedance of a subject's body using a series of electrodes placed on the skin surface. Changes in electrical impedance at the body's surface are used to determine parameters, such as changes in fluid levels, associated with the cardiac cycle or oedema.

US2006/0111652 describes methods for enhancing blood and lymph flow in the extremities of a human. As part of this method, impedance measurements are used to assess segmental blood flows within the limbs.

US2005/0177062 describes a system for measuring the volume, composition and the movement of electroconductive body fluids, based on the electrical impedance of the body or a body segment. This is used primarily for electromechanocardiography (ELMEC) or impedance cardiography (IKG) measurements for determining hemodynamic parameters.

WO00/79255 describes a method of detection of oedema by measuring bioelectrical impedance at two different anatomical regions in the same subject at a single low frequency alternating current. The two measurements are analysed to obtain an indication of the presence of tissue oedema by comparing with data obtained from a normal population.

Co-pending application PCT/AU09/000163 describes a method and apparatus for use in analysing impedance measurements, and in particular, a method and apparatus for determining an indicator indicative of extracellular fluid levels using impedance measurements, the indicator being usable in identifying venous insufficiency, lymphoedema and/or oedema. A method according to the preamble of claim 1 is known from the article by H. Scharfetter et al. "Effect of postural changes on the reliability of volume estimations from bioimpedance spectroscopy data", Kidney International, Vol. 51 (1997), pages 1078-1087.

**Summary of the Present Invention**

A method according to the invention is defined in claim 1.

Typically the indicator is indicative of a change between the at least one first impedance value and the at least one second impedance value.

Typically the method includes, in the processing system:

a) comparing the indicator to a reference; and,
b) providing an indication of the results of the comparison to allow determination of a presence, absence or degree of venous insufficiency.

Typically the method includes, in the processing system:

a) determining the at least one first impedance value with the subject in a first orientation; and
b) determining the at least one second impedance value with the subject in a second orientation.

Typically the method includes, in the processing system:

a) determining the at least one first impedance value with the subject in a first orientation; and,
b) after positioning the subject in a second orientation for a predetermined time period, determining the at least one second impedance value with the subject in the first orientation.

Typically the method includes:

a) positioning the subject in a first orientation for a predetermined time period; and,
b) positioning the subject in a second orientation;
and wherein the method further includes, in the processing system:
c) determining the at least one first and second impedance values with the subject in the second orientation.

Typically the torso of the subject remains in a constant orientation, and when the subject is in the first orientation a first leg of the subject is positioned in a first position, and when the subject is in the second orientation the first leg of the subject is positioned in a second position.

Typically the method includes, in the processing system:

a) determining a plurality of impedance values with the subject in a single orientation; and,
b) determining the indicator based on the plurality of impedance values.

Typically the method includes, in the processing system, examining at least one change in the impedance values over time, the at least one change in the impedance values being used in the assessment of venous insufficiency.

The method includes, in the processing system, examining a rate of change in the impedance values over time, the rate of change being usable in the assessment of venous insufficiency.

Typically the method includes, in the processing system, using the rate of change in the assessment of venous insufficiency by determining whether the rate of change is at least one of:

a) constant;
b) non-constant; and,
c) logarithmic.

Typically the method includes, in the processing system:

a) comparing the rate of change to a reference; and,
b) providing an indication of the results of the comparison to allow determination of a presence, absence or degree of venous insufficiency.

Typically the method includes, in the processing system:

a) determining the at least one first impedance value using a plurality of impedance measurements performed at a plurality of different frequencies; and,
b) determining the at least one second impedance value using a plurality of impedance measurements performed at a plurality of different frequencies.

Typically at least one impedance measurement is measured at a measurement frequency of at least one of:

a) less than 100 kHz;
b) less than 50 kHz; and,
c) less than 10 kHz.

Typically the method includes, in the processing system, using the at least one impedance measurement as an estimate of a resistance of the subject at a zero measurement frequency.
Typically at least one impedance measurement is measured at a measurement frequency of at least one of:

a) greater than 200 kHz;
b) greater than 500 kHz; and,
c) greater than 1000 kHz.

Typically the method includes, in the processing system, using the at least one impedance measurement as an estimate of a resistance of the subject at an infinite measurement frequency.
Typically the at least one first and second impedance values are based on impedance parameter values.
Typically the method includes, in the processing system:

a) determining a plurality of impedance measurements; and,
b) determining at least one impedance parameter value from the plurality of impedance measurements.

Typically the impedance parameter values include at least one of:

$R_0$ which is the resistance at zero frequency;
$R_\infty$ which is the resistance at infinite frequency; and,
$Z_c$ which is the resistance at a characteristic frequency.

Typically the method includes, in the processing system, determining the parameter values using the equation:

$$Z = R_\infty + \frac{R_0 - R_\infty}{1 + (j\omega\tau)^{(1-\alpha)}}$$

where:

Z is the measured impedance at angular frequency $\omega$,
$\tau$ is a time constant, and
$\alpha$ has a value between 0 and 1.

Typically the method includes, in the processing system:

a) determining values for impedance parameters $R_0$ and $R_\infty$ from the measured impedance values; and,
b) calculating a value for impedance parameter $R_i$ which is the resistance of intracellular fluid, using the equation:

$$R_i = \frac{R_0 R_\infty}{R_0 - R_\infty}$$

Typically the method includes, in the processing system, determining the indicator using at least one of the equations:

$$I = \frac{\Delta R_i}{\Delta R_e}$$

$$I = \Delta R_i$$

$$I = \frac{d(R_i/R_e)}{dt}$$

$$I = \frac{dR_i}{dt}$$

where:

$I$ is the indicator

$\Delta R_i$ is a change in the resistance of intracellular fluid, and

$\Delta R_e$ is a change in the resistance of intracellular fluid, with $R_e = R_0$.

Typically the method includes, in the processing system, causing the impedance measurements to be performed.

Typically the method includes, in the processing system:

a) causing one or more electrical signals to be applied to the subject using a first set of electrodes;
b) measuring electrical signals across a second set of electrodes applied to the subject in response to the applied one or more signals; and,
c) determining from the applied signals and the measured signals at least one measured impedance value.

Typically the indicator is used in the assessment of venous insufficiency.

An apparatus according to the invention is defined in claim 13.

Typically the apparatus includes a processing system for:

a) causing one or more electrical signals to be applied to the subject using a first set of electrodes;
b) measuring electrical signals across a second set of electrodes applied to the subject in response to the applied one or more signals; and,
c) determining from the applied signals and the measured signals at least one measured impedance value.

Typically the apparatus includes:

a) a signal generator for generating electrical signals; and,
b) a sensor for sensing electrical signals.

**Brief Description of the Drawings**

An example of the present invention will now be described with reference to the the accompanying drawings, in which: -

Figure 1 is a schematic diagram of a first example of impedance measuring apparatus;
Figure 2 is a flowchart of an example of a process for use in analysing impedance measurements;
Figure 3 is a schematic diagram of a second example of impedance measuring apparatus;
Figure 4 is a schematic diagram of an example of a computer system;
Figure 5 is a flowchart of an example of a process for performing impedance measurements;
Figure 6A is a schematic of an example of a theoretical equivalent circuit for biological tissue;

Figure 6B is an example of a locus of impedance known as a Wessel plot;

Figure 7 is a flowchart of a first specific example of a process for analysing impedance measurements to allow assessment of venous insufficiency;

Figure 8 is a flowchart of a second specific example of a process for analysing impedance measurements to allow assessment of venous insufficiency;

Figure 9 is a flowchart of a third specific example of a process for analysing impedance measurements to allow assessment of venous insufficiency;

Figure 10A is an example plot of the changes in intracellular resistance after a change in orientation from a standing position to a supine position in a normal subject;

Figure 10B is an example plot of the changes in intracellular resistance after a change in orientation from a standing position to a supine position in a subject with venous insufficiency;

Figure 10C is an example plot of the changes in extracellular resistance after a change in orientation from a standing position to a supine position in a normal subject, and in a subject with lymphoedema; and,

Figure 10D is an example plot of the changes in extracellular resistance after a change in orientation from a standing position to a supine position in a subject with venous insufficiency.

## Detailed Description of the Preferred Embodiments

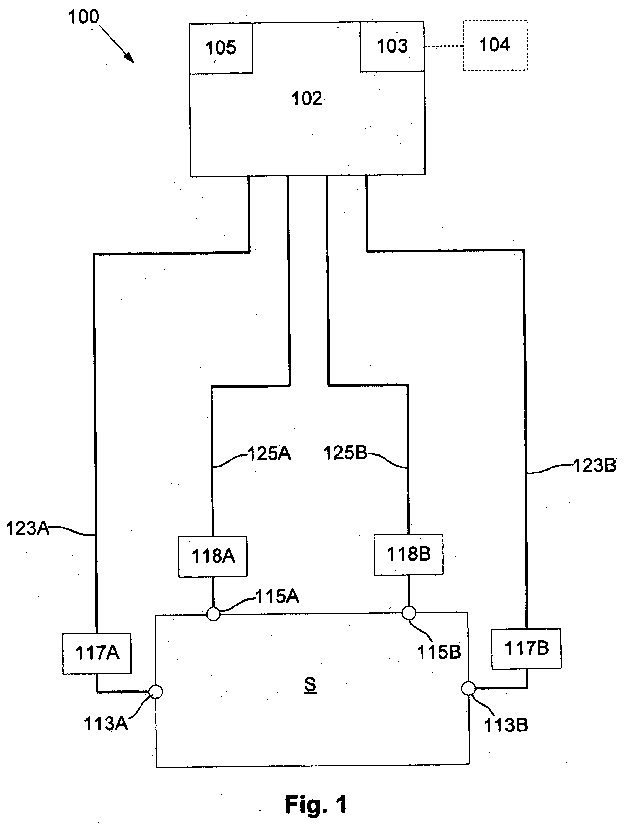

An example of apparatus suitable for performing an analysis of a subject's bioelectric impedance will now be described with reference to Figure 1.

As shown the apparatus includes a measuring device 100 including a processing system 102, connected to one or more signal generators 117A, 117B, via respective first leads 123A, 123B, and to one or more sensors 118A, 118B, via respective second leads 125A, 125B. The connection may be via a switching device, such as a multiplexer, although this is not essential.

In use, the signal generators 117A, 117B are coupled to two first electrodes 113A, 113B, which therefore act as drive electrodes to allow signals to be applied to the subject S, whilst the one or more sensors 118A, 118B are coupled to the second electrodes 115A, 115B, which therefore act as sense electrodes, to allow signals induced across the subject S to be sensed.

The signal generators 117A, 117B and the sensors 118A, 118B may be provided at any position between the processing system 102 and the electrodes 113A, 113B, 115A, 115B, and may therefore be integrated into the measuring device 100.

However, in one example, the signal generators 117A, 117B and the sensors 118A, 118B are integrated into an electrode system, or another unit provided near the subject S, with the leads 123A, 123B, 125A, 125B connecting the signal generators 117A, 117B and the sensors 118A, 118B to the processing system 102. By performing this, the length of any connections between the signal generators 117A, 117B and the sensors 118A, 118B, and the corresponding electrodes 113A, 113B, 115A, 115B can be reduced. This minimises any parasitic capacitances between the connections, the connections and the subject, and the connections and any surrounding articles, such as a bed on which the subject is provided, thereby reducing measurement errors.

The above described system can be described as a two channel device, with each channel being designated by the suffixes A, B respectively. The use of a two channel device is for the purpose of example only, and any number of channels may be provided, as required.

An optional external interface 103 can be used to couple the measuring device 100, via wired, wireless or network connections, to one or more peripheral devices 104, such as an external database or computer system, barcode scanner, or the like. The processing system 102 will also typically include an I/O device 105, which may be of any suitable form such as a touch screen, a keypad and display, or the like.

In use, the processing system 102 is adapted to generate control signals, which cause the signal generators 117A, 117B to generate one or more alternating signals, such as voltage or current signals of an appropriate waveform, which can be applied to a subject S, via the first electrodes 113A, 113B. The sensors 118A, 118B then determine the voltage across or current through the subject S, using the second electrodes 115A, 115B and transfer appropriate signals to the processing system 102.

Accordingly, it will be appreciated that the processing system 102 may be any form of processing system which is suitable for generating appropriate control signals and at least partially interpreting the measured signals to thereby determine the subject's bioelectrical impedance, and optionally determine other information such indicators of the presence, absence or degree of venous insufficiency, other conditions, or the like.

The processing system 102 may therefore be a suitably programmed computer system, such as a laptop, desktop, PDA, smart phone or the like. Alternatively the processing system 102 may be formed from specialised hardware, such as an FPGA (field programmable gate array), or a combination of a programmed computer system and specialised hardware, or the like, as will be described in more detail below.

In use, the first electrodes 113A, 113B are positioned on the subject to allow one or more signals to be injected into the subject S. The location of the first electrodes will depend on the segment of the subject S under study. Thus, for example, the first electrodes 113A, 113B can be placed on the thoracic and neck region of the subject S to allow the impedance of the chest cavity to be determined for use in cardiac function analysis. Alternatively, positioning electrodes on the wrist and ankles of a subject allows the impedance of limbs and/or the entire body to be determined, for use in oedema analysis, assessment of venous insufficiency, or the like.

Once the electrodes are positioned, one or more alternating signals are applied to the subject S, via the first electrodes 113A, 113B. The nature of the alternating signal will vary depending on the nature of the measuring device and the subsequent analysis being performed.

For example, the system can use Bioimpedance Analysis (BIA) in which a single low frequency signal is injected into the subject S, with the measured impedance being used directly in the determination of biological parameters, such as extracellular fluid levels, which can be indicative of oedema, and hence of venous insufficiency.

In one example, the applied signal has a relatively low frequency, such as below 100 kHz, more typically below 50 kHz and more preferably below 10 kHz. In this instance, such low frequency signals can be used as an estimate of the impedance at zero applied frequency, commonly referred to as the impedance parameter value $R_0$, which is in turn indicative of extracellular fluid levels.

Alternatively, the applied signal can have a relatively high frequency, such as above 200 kHz, and more typically above 500 kHz, or 1000 kHz. In this instance, such high frequency signals can be used as an estimate of the impedance at infinite applied frequency, commonly referred to as the impedance parameter value $R_\infty$, which is in turn indicative of a combination of the extracellular and intracellular fluid levels, as will be described in more detail below.

A parameter indicative of intracellular fluid levels alone can also be determined if values of the impedance parameter values $R_0$ and $R_\infty$ are both obtained, as will be described below.

In contrast Bioimpedance Spectroscopy (BIS) devices perform impedance measurements at multiple frequencies over a selected frequency range. Whilst any range of frequencies may be used, typically frequencies range from very low frequencies (4 kHz) to higher frequencies (15000 kHz). Similarly, whilst any number of measurements may be made, in one example the system can use 256 or more different frequencies within this range, to allow multiple impedance measurements to be made within this range.

When impedance measurements are made at multiple frequencies, these can be used to derive one or more impedance parameter values, such as values of $\alpha$, $R_0$, $Z_c$, $R_\infty$, which correspond to the dispersion width of the impedance measurements, and the impedance at zero, characteristic and infinite frequencies respectively. These can in turn be used to determine information regarding intracellular and/or extracellular fluid levels, as will be described in more detail below.

A further alternative is for the system to use Multiple Frequency Bioimpedance Analysis (MFBIA) in which multiple signals, each having a respective frequency are injected into the subject S, with the measured impedances being used in the assessment of fluid levels. In one example, four frequencies can be used, with the resulting impedance measurements at each frequency being used to derive impedance parameter values, for example by fitting the measured impedance values to a Cole model, as will be described in more detail below. Alternatively, the impedance measurements at each frequency may be used individually or in combination.

Thus, the measuring device 100 may either apply an alternating signal at a single frequency, at a plurality of frequencies simultaneously, or a number of alternating signals at different frequencies sequentially, depending on the preferred implementation. The frequency or frequency range of the applied signals may also depend on the analysis being performed.

In one example, the applied signal is generated by a voltage generator, which applies an alternating voltage to the subject S, although alternatively current signals may be applied.

In one example, the voltage source is typically symmetrically and/or differentially arranged, with each of the signal generators 117A, 117B being independently controllable, to allow the potential across the subject to be varied. This can be performed to reduce the effects of any imbalance, which occurs when the voltages sensed at the electrodes are unsymmetrical (a situation referred to as an "imbalance"). In this instance, any difference in the magnitude of signals within the leads can lead to differing effects due to noise and interference.

Whilst applying the voltage symmetrically, can reduce the effect, this is not always effective if the electrode impedances for the two drive electrodes 113A, 113B are unmatched, which is typical in a practical environment. However, by adjusting the differential drive voltages applied to each of the drive electrodes 113A, 113B, this compensates for the different electrode impedances, and restores the desired symmetry of the voltage at the sense electrodes 115A, 115B. This can be achieved by measuring the voltages at the sense electrodes, and then adjusting the magnitude and/or phase of the applied signal to thereby balance the magnitude of the sensed voltages. This process is referred to herein as *balancing* and in one example is performed by minimizing the magnitude of any common mode signal.

A potential difference and/or current is measured between the second electrodes 115A, 115B. In one example, the voltage is measured differentially, meaning that each sensor 118A, 118B is used to measure the potential at each second electrode 115A, 115B and therefore need only measure half of the potential as compared to a single ended system.

The acquired signal and the measured signal will be a superposition of potentials generated by the human body, such as the ECG (electrocardiogram), potentials generated by the applied signal, and other signals caused by environmental electromagnetic interference. Accordingly, filtering or other suitable analysis may be employed to remove unwanted components.

The acquired signal is typically demodulated to obtain the impedance of the system at the applied frequencies. One suitable method for demodulation of superposed frequencies is to use a Fast Fourier Transform (FFT) algorithm to transform the time domain data to the frequency domain. This is typically used when the applied current signal is a superposition of applied frequencies. Another technique not requiring windowing of the measured signal is a sliding window FFT.

In the event that the applied current signals are formed from a sweep of different frequencies, then it is more typical to use a signal processing technique such as correlating the signal. This can be achieved by multiplying the measured signal with a reference sine wave and cosine wave derived from the signal generator, or with measured sine and cosine waves, and integrating over a whole number of cycles. This process, known variously as quadrature demodulation or synchronous detection, rejects all uncorrelated or asynchronous signals and significantly reduces random noise.

Other suitable digital and analogue demodulation techniques will be known to persons skilled in the field.

In the case of BIS, impedance or admittance measurements can be determined from the signals at each frequency using the recorded voltage across and current flow through the subject. The demodulation algorithm can then produce an amplitude and phase signal at each frequency. This can then be used to derive one or more impedance parameter values, if required.

As part of the above described process, the position of the second electrodes may be measured and recorded. Similarly, other parameters relating to the subject (subject parameters) may be recorded, such as the height, weight, age, sex, health status, any interventions and the date and time on which they occurred. Other information, such as current medication, may also be recorded. This can then be used in performing further analysis of the impedance measurements, so as to allow determination of the presence, absence or degree of venous insufficiency, oedema, lymphoedema, or the like.

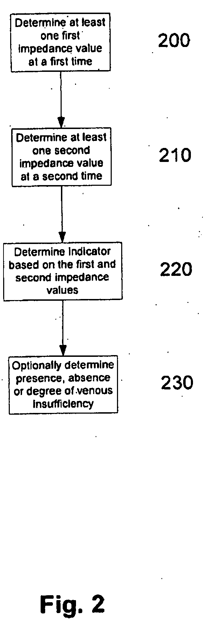

An example of the process of analysing impedance measurements and the operation of the apparatus of Figure 1 to perform this will now be described with reference to Figure 2.

At step 200, at least one first impedance value indicative of the impedance of at least one segment of the subject's leg is determined at a first time. This may be achieved by having the signal generators 117A, 117B, apply at least one first signal to the subject S, via the first electrodes 113A, 113B, with second signals being measured across the subject S by the sensors 118A, 118B, via the second electrodes 115A, 115B. An indication of the first and second signals is provided to the processing system 102, allowing the impedance, or an impedance parameter value to be determined. The leg segment may be any suitable segment of the leg for which changes in fluid levels can be measured, but is typically a segment of the lower leg or calf region.

At step 210, at least one second impedance value indicative of the impedance of the at least one segment of the subject's leg is determined at a second time, using similar methods as for the first impedance value.

Throughout this process, the subject is arranged such that a body fluid level in the at least one segment of the subject's leg changes between the times that the first and second impedance values are determined. It will be appreciated that this can be performed in a number of ways.

For example, the orientation of all or part of the subject can be changed after the determining the first impedance value so that the second impedance value will be indicative of the impedance of the at least one segment of the subject's leg for a different orientation to that of the first impedance value. Body fluid levels will redistribute as a result of the change in orientation and this will result in a body fluid level change between the times that the first and second impedance values are determined.

Alternatively, a body fluid level change between measurements can be caused by changing the orientation of the subject before the impedance values are determined, promoting the flow of body fluids into or out of the segment of the subject's leg, depending on the nature of the orientation change, and therefore resulting in first and second impedance values being determined for different body fluid levels.

Procedures for causing changes in the body fluid level in the subject will be described in the further detailed examples in this specification below.

At step 220, an indicator is determined based on the at least one first and at least one second impedance values. The indicator is typically indicative of a change between the at least one first impedance value and the at least one second impedance value, and hence indicative of the change in body fluid levels between measurements.

Typically, the indicator is at least partially indicative of at least the intracellular and/or extracellular fluid levels in the at least one segment of the subject's leg. Generally, indicators are derived using multiple measurements due to the electrical properties of the body segment, as will be described in further detail below. Accordingly, in one example, the first and second impedance values are determined using multiple measurements performed at multiple frequencies, with the respective impedance values being based on appropriate impedance parameter values derived therefrom, such as the impedance at zero applied frequency $R_0$, and impedance at infinite applied frequency $R_\infty$, as will be described in more detail below. Other impedance parameters may be used, for example, a dispersion parameter such as a which represents a distribution of the impedance measurements about an ideal model.

In one example, the indicator is indicative of a change in the body fluid levels over time, and in another example, the indicator is indicative of a rate of change in the body fluid levels over successive measurements. Alternatively, the indicator can be indicative of a ratio of changes in intracellular fluid levels to changes in extracellular fluid levels. It will be appreciated that the indicator can be determined and represented in a number of ways, and further examples will be described in more detail below.

At step 230, the indicator can optionally be used in the assessment of venous insufficiency, or other conditions, such as oedema or lymphoedema. In this regard, the properties of the changes in fluid levels in the leg segment after an orientation change, which are indicated by the indicator, can be used to determine whether venous insufficiency may be present. In one example, the indicator can be compared to a reference, such as a normal population reference, to allow the presence, absence or degree of venous insufficiency to be determined, as will be described in more detail below.

The magnitude or rate of change in responsiveness of body fluid levels after orientation changes is a good indicator for venous insufficiency because the nature of the body fluid level changes as a result of an orientation change are different for normal subjects and subjects with venous insufficiency.

Specifically, if a subject is moved from a position that promotes maximum blood pooling in the legs, such as standing or sitting with the lower leg portions unsupported, to a position that de-loads the leg, such as a supine position or sitting with the lower leg portions elevated, different types of changes in the body fluid levels occurs for venous insufficiency subjects compared to normal subjects.

For example, the body fluid level changes in a venous insufficiency subject are typically greater than for a normal subject shortly after an orientation change from a standing position to a supine position, as the blood that was pooled in the leg due to malfunctioning valves in the veins of the venous insufficiency subject will rapidly flow out of the leg when the influence of gravity is reduced. Similarly, in the reverse situation in which a subject changes orientation from a supine position to a standing position, more rapid pooling will occur in the venous insufficiency subject compared to a normal subject.

By comparing the characteristics of the changes of the subject to reference characteristics taken from a population of normal or venous insufficiency subjects, the presence, absence or degree of venous insufficiency can be effectively determined.

The amount of blood pooling can be indicated by measurements of extracellular fluid alone, with the extracellular fluid levels being indicative of blood volume within the leg segment.

Thus, for example, a high extracellular impedance is indicative of a low volume of blood, so as the extracellular impedance increases, this indicates a reduction in blood pooling. Consequently, measuring changes in the extracellular impedance can be used to determine the rate of blood pooling and hence the presence, absence or degree of venous insufficiency.

Additionally, the intracellular impedance is also indirectly influenced by subject orientation, caused by changes in blood cell orientation. In this regard, when standing, the blood cells are typically aligned, resulting in a high intracellular impedance, whilst in a supine position, the cells align randomly, resulting in a reduced intracellular impedance. This effect is again exacerbated in a subject suffering from venous insufficiency, as compared to a normal subject, due to differences in the rate and degree of blood pooling.

Initial studies on control patients measuring the impedance during positional changes have shown that while the extracellular component of the impedance changes as expected during filling and return, the intracellular component of the impedance behaves in the opposite manner. That is, as pooling increases, the intra-cellular fluid measured decreases. This is predicted to be due to gravitational settling of the cells. As the cells settle, they will align with a minimal cross sectional area facing the direction of the applied current. This induces a decrease in the cross sectional area captured by impedance measurements and therefore an increase in impedance during venous filling, despite the increase in the number of cells and therefore the amount of intra-cellular fluid.

It will be appreciated from the above that the change in intracellular and extracellular impedances can be used either alone, or in combination.

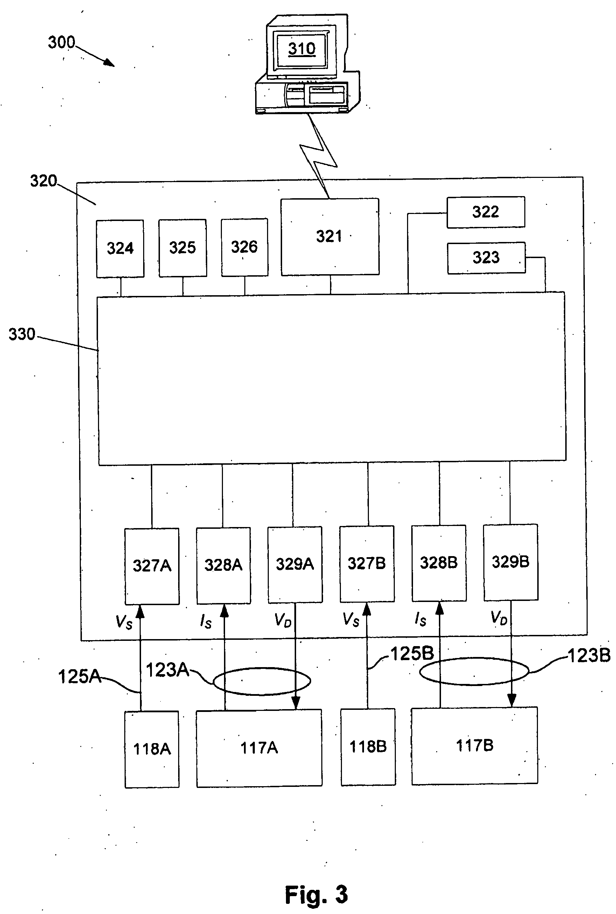

A specific example of the apparatus will now be described in more detail with respect to Figure 3.

In this example, the measuring system 300 includes a computer system 310 and a separate measuring device 320. The measuring device 320 includes a processing system 330 coupled to an interface 321 for allowing wired or wireless communication with the computer system 310. The processing system 330 may also be optionally coupled to one or more stores, such as different types of memory, as shown at 322, 323, 324, 325, 326.

In one example, the interface is a Bluetooth stack, although any suitable interface may be used. The memories can include a boot memory 322, for storing information required by a boot-up process, and a programmable serial number memory 323, that allows a device serial number to be programmed. The memory may also include a ROM (Read Only Memory) 324, flash memory 325 and EPROM (Electronically Programmable ROM) 326, for use during operation. These may be used for example to store software instructions and to store data during processing, as will be appreciated by persons skilled in the art.

A number of analogue to digital converters (ADCs) 327A, 327B, 328A, 328B and digital to analogue converters (DACs) 329A, 329B are provided for coupling the processing system 330 to the sensors 118A, 118B and the signal generators 117A, 117B, as will be described in more detail below.

A controller, such as a microprocessor, microcontroller or programmable logic device, may also be provided to control activation of the processing system 330, although more typically this is performed by software instructions executed by the processing system 330.

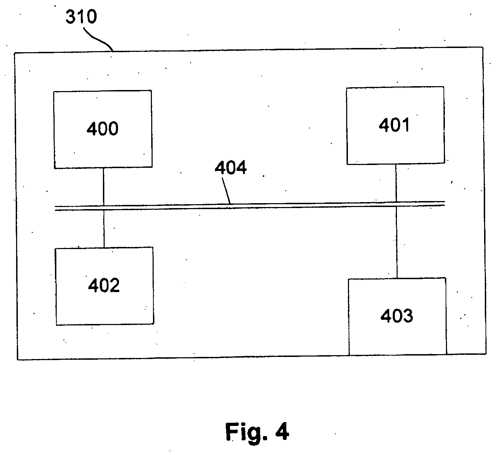

An example of the computer system 310 is shown in Figure 4. In this example, the computer system 310 includes a processor 400, a memory 401, an input/output device 402 such as a keyboard and display, and an external interface 403 coupled together via a bus 404, as shown. The external interface 403 can be used to allow the computer system to communicate with the measuring device 320, via wired or wireless connections, as required, and accordingly, this may be in the form of a network interface card, Bluetooth stack, or the like.

In use, the computer system 310 can be used to control the operation of the measuring device 320, although this may alternatively be achieved by a separate interface provided on the measuring device 300. Additionally, the computer system 310 can be used to allow at least part of the analysis of the impedance measurements to be performed.

Accordingly, the computer system 310 may be formed from any suitable processing system, such as a suitably programmed PC, Internet terminal, lap-top, hand-held PC, smart phone, PDA, server, or the like, implementing appropriate applications software to allow required tasks to be performed.

In contrast, the processing system 330 typically performs specific processing tasks, to thereby reduce processing requirements on the computer system 310. Thus, the processing system typically executes instructions to allow control signals to be generated for controlling the signal generators 117A, 117B, as well as the processing to determine instantaneous impedance values.

In one example, the processing system 330 is formed from custom hardware, or the like, such as a Field Programmable Gate Array (FPGA), although any suitable processing module, such as a magnetologic module, may be used.

In one example, the processing system 330 includes programmable hardware, the operation of which is controlled using instructions in the form of embedded software instructions. The use of programmable hardware allows different signals to be applied to the subject S, and allows different analysis to be performed by the measuring device 320. Thus, for example, different embedded software would be utilised if the signal is to be used to analyse the impedance at a number of frequencies simultaneously as compared to the use of signals applied at different frequencies sequentially.

The embedded software instructions used can be downloaded from the computer system 310. Alternatively, the instructions can be stored in memory such as the flash memory 325 allowing the instructions used to be selected using either an input device provided on the measuring device 320, or by using the computer system 310. As a result, the computer system 310 can be used to control the instructions, such as the embedded software, implemented by the processing system 330, which in turn alters the operation of the processing system 330.

Additionally, the computer system 310 can operate to analyse impedance determined by the processing system 330, to allow biological parameters to be determined.

Whilst an alternative arrangement with a single processing system may be used, the division of processing between the computer system 310 and the processing system 330 can provide some benefits.

Firstly, the use of the processing system 330 more easily allows the custom hardware configuration to be adapted through the use of appropriate embedded software. This in turn allows a single measuring device to be used to perform a range of different types of analysis.

Secondly, the use of a custom configured processing system 330 reduces the processing requirements on the computer system 310. This in turn allows the computer system 310 to be implemented using relatively straightforward hardware, whilst still allowing the measuring device to perform sufficient analysis to provide interpretation of the impedance. This can include for example generating a "Wessel" plot, using the impedance values to determine parameters relating to cardiac function, as well as determining the presence or absence of lymphoedema.

Thirdly, this allows the measuring device 320 to be updated. Thus for example, if an improved analysis algorithm is created, or an improved current sequence determined for a specific impedance measurement type, the measuring device can be updated by downloading new embedded software via flash memory 325 or the external interface 321.

In use, the processing system 330 generates digital control signals, which are converted to analogue voltage drive signals $V_D$ by the DACs 329, and transferred to the signal generators 117. Analogue signals representing the current of the drive signal $I_D$ applied to the subject and the subject voltage $V_S$ measured at the second electrodes 115A, 115B are received from the signal generators 117 and the sensors 118 and are digitised by the ADCs 327, 328. The digital signals can then be returned to the processing system 330 for preliminary analysis.

In this example, a respective set of ADCs 327, 328, and DACs 329 are used for each of two channels, as designated by the reference numeral suffixes A, B respectively. This allows each of the signal generators 117A, 117B to be controlled independently and for the sensors 118A, 118B to be used to detect signals from the electrodes 115A, 115B respectively. This therefore represents a two channel device, each channel being designated by the reference numerals A, B.

In practice, any number of suitable channels may be used, depending on the preferred implementation. Thus, for example, it may be desirable to use a four channel arrangement, in which four drive and four sense electrodes are provided, with a respective sense electrode and drive electrode pair being coupled to each limb. In this instance, it will be appreciated that an arrangement of eight ADCs 327, 328, and four DACs 329 could be used, so each channel has respective ADCs 327, 328, and DACs 329. Alternatively, other arrangements may be used, such as through the inclusion of a multiplexing system for selectively coupling a two-channel arrangement of ADCs 327, 328, and DACs 329 to a four channel electrode arrangement, as will be appreciated by persons skilled in the art.

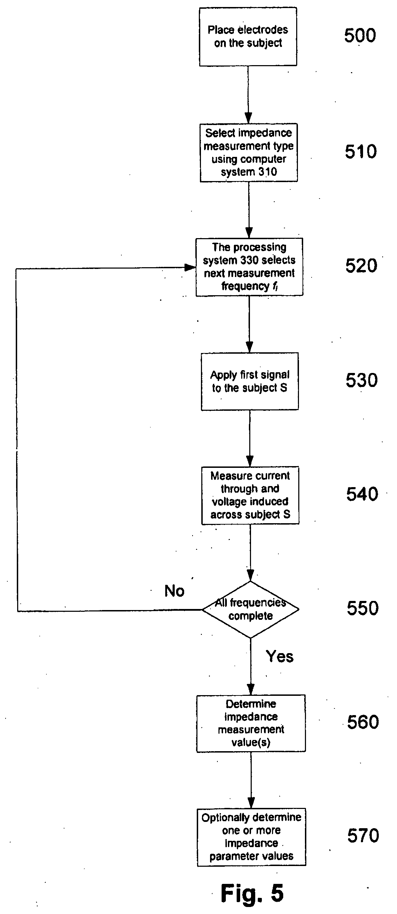

An example of the process for performing impedance measurements will now be described with reference to Figure 5.

At step 500, the electrodes are positioned on the subject as required. The general arrangement to allow impedance of a leg to be determined is to provide drive electrodes 113A, 113B on the hand at the base of the knuckles and on the feet at the base of the toes, on the side of the body being measured. Sense electrode 115A are also positioned at the front of the ankle on the leg being measured, with the sense electrode 115B being positioned anywhere on the contra-lateral leg.

It will be appreciated that this configuration uses the theory of equal potentials, allowing the electrode positions to provide reproducible results for impedance measurements. This is advantageous as it greatly reduces the variations in measurements caused by poor placement of the electrodes by the operator.

Alternatively however other arrangements can be used. Thus for example, the sense electrodes can be provided anywhere on the leg of interest, allowing the impedance measurements to be made along the entire leg, or for a part of the leg (generally referred to as a leg segment), such as a calf segment, or the like.

At step 510, an impedance measurement type is selected using the computer system 310, allowing the computer system 310 to determine an impedance measurement protocol, and configure the processing system 330 accordingly. This is typically achieved by configuring firmware or software instructions within the processing system 330, as described above.

At step 520, the processing system 300 selects a next measurement frequency $f_i$, and causes the signal generators 117A, 117B to apply a first signal to the subject at the selected frequency at step 530. At step 540, the signal generators 117A, 117B and sensors 118A, 118B provide an indication of the current through and the voltage across the leg segment to the processing system 330.

At step 550, the processing system 330 determines if all frequencies are complete, and if not returns to step 520 to select the next measurement frequency. At step 560, one or more measured impedance values are determined, by the computer system 310, the processing system 330, or a combination thereof, using the techniques described above. One or more impedance parameter values may optionally be derived at step 570.

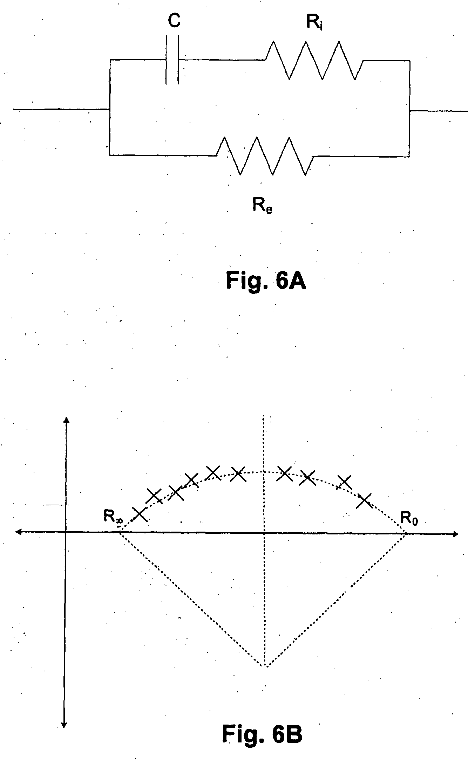

In this regard, Figure 6A is an example of an equivalent circuit that effectively models the electrical behaviour of biological tissue. The equivalent circuit has two branches that represent current flow through extracellular fluid and intracellular fluid, respectively. The extracellular fluid component of biological impedance is represented by an extracellular resistance $R_e$, whilst the intracellular fluid component is represented by an intracellular resistance $R_i$ and a capacitance C representative of the cell membranes.

The relative magnitudes of the extracellular and intracellular components of impedance of an alternating current (AC) are frequency dependent. At zero frequency the capacitor acts as a perfect insulator and all current flows through the extracellular fluid, hence the resistance at zero frequency, $R_0$, equals the extracellular resistance $Re$. At infinite frequency the capacitor acts as a perfect conductor and the current passes through the parallel resistive combination. The resistance at infinite frequency $R_\infty$ is given by:

$$R_\infty = \frac{R_e R_i}{R_e + R_i} \tag{1}$$

Accordingly, the impedance of the equivalent circuit of Figure 6A at an angular frequency $\omega$, where ($\omega=2\pi$*frequency, is given by:

$$Z = R_\infty + \frac{R_0 - R_\infty}{1 + (j\omega\tau)} \tag{2}$$

where:

$R_\infty$ = impedance at infinite applied frequency
$R_0$ = impedance at zero applied frequency = $R_e$ and,
$\tau$ is the time constant of the capacitive circuit.

However, the above represents an idealised situation which does not take into account the fact that the cell membrane is an imperfect capacitor. Taking this into account leads to a modified model in which:

$$Z = R_\infty + \frac{R_0 - R_\infty}{1 + (j\omega\tau)^{(1-\alpha)}} \qquad (3)$$

where:

$\alpha$ is a dispersion parameter which can be thought of as an indicator of the deviation of a real system from the ideal model and has a value between 0 and 1.

The values of impedance parameters a, $R_0$, $R_\infty$ or $Z_c$ may be determined in any one of a number of manners such as by:

- estimating values based on impedance measurements performed at selected respective frequencies;
- solving simultaneous equations based on the impedance values determined at different frequencies;
- using iterative mathematical techniques;
- extrapolation from a "Wessel plot" similar to that shown in Figure 6B;
- performing a function fitting technique, such as the use of a polynomial function.

For example, the Wessel plot is often used in BIS (Bioimpedance Spectroscopy) Bioimpedance Spectroscopy (BIS) devices, which perform multiple measurements over a range of frequencies, such as from 4 kHz to 1000 kHz, using 256 or more different frequencies within this range. A regression procedure is then used to fit the measured data to the theoretical semi-circular locus, allowing values for $R_\infty$ and $R_0$ to be calculated.

Such a regression analysis is computationally expensive, typically requiring a larger or more expensive device. The regression analysis and also requires a large number of data points, which can cause the measurement process to take a significant amount of time.

Alternatively, a circle technique can be used in which only three measurement points are required. In this technique, three simultaneous equations representing the geometric relationships between points on a circle are solved to allow calculation of the radius (r) and the co-ordinates of the centre of the circle (i, j) as the three parameters which define the circle. From these circle parameters, $R_0$ and $R_\infty$ are readily computed from geometric first principles.

This circle technique allows a value for $R_0$ and $R_\infty$ to be derived in a computationally less expensive manner than if a regression analysis is performed, and requires a reduced number of data points allowing a more rapid measurement process.

One potential disadvantage of the use of simultaneous equations is that if one of the impedance measurements is inaccurate for any reason, this can lead to large deviations in the calculated values of $R_0$ and/or $R_\infty$. Accordingly, in one example, impedance measurements are performed at more than three frequencies, with circle parameters for all possible combinations of impedance measurements at three frequencies being calculated. The average can be provided along with the standard deviation as a measure of the goodness of fit of the data to the Cole model. In the event that one of the measurements is inaccurate, this can be accounted for by excluding one or more outlier measurements, such as measurements that deviates the greatest amount from the mean, or measurements differing by more than a set number of standard deviations from the mean, allowing the mean to be recalculated, thereby providing more accurate values.

Whilst this process uses additional measurements, such as four or five measurements, this is still significantly less than the 256 or more frequencies typically performed using a BIS measurement protocol, allowing the measurement process to be performed more quickly.

In one example, the frequencies used are in the range 0 kHz to 1000 kHz, and in one specific example, four measurements are recorded at frequencies of 25 kHz, 50 kHz, 100 kHz, and 200 kHz, although any suitable measurement frequencies can be used.

A further alternative for determining impedance parameter values such as $R_0$ and $R_\infty$ is to perform impedance measurements at a single frequency, and use these as an estimate of the parameter values. In this instance, measurements performed at a single low frequency can be used to estimate $R_0$, whilst measurements at a single high frequency can be used to estimate $R_\infty$.

The above described equivalent circuit models the resistivity as a constant value and does not therefore accurately reflect the impedance response of a subject, and in particular does not accurately model the change in orientation of the erythrocytes in the subject's blood stream, or other relaxation effects. To more successfully model the electrical conductivity of the human body, an improved CPE based model may alternatively be used.

In any event, it will be appreciated that any suitable technique for determination of the parameter values such as $R_0$, $Z_c$, $R_\infty$, and $\alpha$ may be used.

It will also be appreciated that determination of the parameter values allows values of the resistances of the extracellular and intracellular body fluid levels to be determined. The value of the resistance of extracellular fluid, $R_e$, is easily determined as it is equal to $R_0$. On the other hand, the value of the resistance of intracellular fluid, $R_i$, is given by:

$$R_i = \frac{R_0 R_\infty}{R_0 - R_\infty} \tag{5}$$

Determination of the parameter values of the body fluid resistances for two or more measurements between which changes in the body fluid levels are induced allows indicators to be derived which are indicative of changes in the fluid levels, which can subsequently be used in the assessment of venous insufficiency.

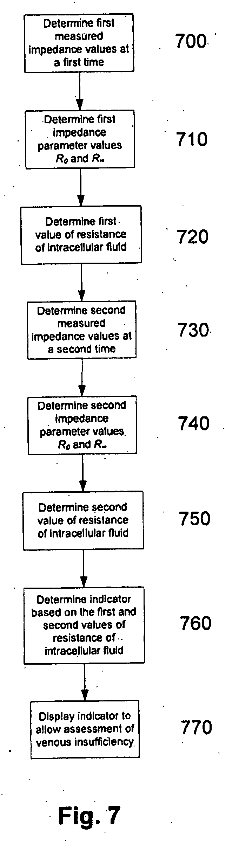

A first specific example of a process for analysing impedance measurements to allow assessment of venous insufficiency will now be described with reference to Figure 7.

In this example, at step 700, at least one first impedance value is determined at a first time using the method described above. The measurement is typically performed with the subject in a specific orientation, such as in a supine or standing position. This is performed to either maximise or minimise the effect of blood pooling, and this will depend on the analysis performed. Depending on the specific protocol, the first measurement can be performed after the subject has been in the specific orientation for a predetermined time, or alternatively after a change in orientation, such as from the standing position to the supine position, to cause the level of blood pooling to be changing when the measurement is performed.

In this first specific example, the subject is made to stand for a set time period such as between five and fifteen minutes to maximize the effect of any blood pooling. In general, a marked increase in blood pooling is achieved after five minutes, with the blood levels reaching a relatively static maximum after approximately fifteen minutes. Accordingly, whilst it is preferable for the subject to stand for fifteen minutes to thereby maximise blood pooling, even after five minutes sufficient pooling occurs to allow measurements to be performed. It will be appreciated from this that the length of time selected may depend on factors such as the amount of time available for the measurement process and the ability of the subject to remain in standing position.

Furthermore, the subject may be required to lay in a supine position for a set time period, such as five to fifteen minutes prior to standing. This can be performed to minimise any blood pooling before standing, so as to provide a more accurate baseline status for the subject prior to measurements being performed. Again, a marked reduction in pooling is achieved after five minutes, with the level of pooling typically reaching a reasonably static minimum after approximately fifteen minutes, so the length of time used will depend on factors such as the amount of time available to make a measurement.

At step 710, first impedance parameter values $R_0$ and $R_\infty$ are optionally determined using the first impedance measurements. This can be performed if three or more impedance values are measured, as previously discussed. Alternatively, approximations of $R_0$ and $R_\infty$ may be determined, using a first single impedance measurement at a low frequency, such as below 10 kHz, to provide a reasonably close approximation of $R_0$, and a second single impedance measurement at a high frequency, such as above 1000 kHz, to provide a reasonably close approximation of $R_\infty$.

At step 720, a first value of the resistance of intracellular fluid $R_{i1}$ in the at least one segment of the subject's leg is determined using the determined first impedance parameter values of $R_0$ and $R_\infty$. It will be appreciated that a first value of the resistance of extracellular fluid $R_{e1}$ is already known at this time as this parameter is equal to $R_0$.

At step 730, at least one second impedance value is determined at a second time in a similar fashion to step 700, typically following a change in the subject's body fluid levels as a result of changing the orientation of the subject either before or after the first measurement. The time between determinations of the at least one first and second impedance values may optionally be recorded.

At step 740, second impedance parameter values $R_0$ and $R_\infty$ are determined using the second impedance measurements, and typically using the same technique as used for the first impedance parameter values at step 710. At step 750, a second value of the resistance of intracellular fluid $R_{i2}$ is determined using the determined second impedance parameter values of $R_0$ and $R_\infty$. Similarly a second value of the resistance of extracellular fluid $R_{e2}$ may also

be determined.

At step 750, an indicator is determined based on the first and second values of resistance of intracellular fluid. In this specific example, the indicator is indicative of a change in the intracellular fluid levels within the subject. The indicator is displayed to the user at step 760 to allow assessment of venous insufficiency or oedema.

The indicator can be any form of suitable indicator such as a numerical value based on the difference between the first and second values of the resistance of intracellular fluid $R_i$. For example, the indicator $I$ may be given by:

$$I = (R_{i1} - R_{i2}) = \Delta R_i \tag{6}$$

In another example, first and second values of the resistance of extracellular fluid $R_e$ are also determined, and the indicator is based on a ratio of the differences between the first and second values of the resistance of intracellular fluid and the differences between the first and second values of the resistance of intracellular fluid. In this example, the indicator $I$ may be given by:

$$I = \frac{(R_{i1} - R_{i2})}{(R_{e1} - R_{e2})} = \frac{\Delta R_i}{\Delta R_e} \tag{7}$$

The indicator may also be scaled to provide a numerical value that is indicative of the presence, absence or degree of venous insufficiency or oedema. The indicator can also be based on the results of a comparison of a numerical value to a reference. The reference could be any suitable form of reference. Thus, in one example, the reference can be based on a reference derived from sample populations, or the like. The reference can be selected based on the subject parameters, so that the value of the indicator is compared to values of the indicator derived from a study of a sample population of other individuals having similar subject parameters.

Alternatively, the reference can be based on a previously measured reference for the subject, for example determined before the subject suffered from venous insufficiency or oedema. This allows a longitudinal analysis to be performed, thereby allowing the onset or progression of venous insufficiency to be assessed.

As a further alternative, the reference can be based on equivalent changes in impedance parameter values determined for a different limb of the subject, such as an arm. This is possible, as, for a subject not suffering from venous insufficiency, there is a predictable relationship between different limbs in changes of intracellular fluid levels as a result of an orientation change. Thus, for example, if the subject is suffering from a condition other than venous insufficiency, which causes a general change in how intracellular fluid levels change over time, then this should affect body segments in an assessable manner, thereby allowing venous insufficiency to be identified.

The indicator can additionally and/or alternatively be displayed on a graphical linear or nonlinear scale, with the position of a pointer on the scale being at least partially indicative of a change in intracellular fluid levels and or the presence, absence or degree of oedema or venous insuffciency. In one example, the linear scale can include thresholds at values representing ranges indicative of the presence or absence of oedema or venous insufficiency, as derived from sample population data, or other references.

At step 770, the user can use the indicator to assess whether further investigation is required. In this regard, a large decrease in intracellular fluid level after a subject is moved from a standing position (maximising blood pooling in the leg segment) to a supine position (deloading the leg segment) is a good indication that the subject has venous insufficiency, but this may need to be confirmed with further measurements, and/or analysis.

The above described example allows for a rapid assessment of the presence of venous insufficiency. This can be performed using BIA, which allows relatively simple apparatus and processing to be used, thereby reducing the cost of equipment required to assess venous insufficiency compared to more complex techniques. Despite this, the process is more reliable than current non-invasive techniques such as SPG and APG. In this regard, changes in fluid levels can typically be detected using impedance measurements before the fluid level changes have a noticeable impact on limb volume, thereby making the impedance measurement process more sensitive than other techniques such as SPG or APG.

In the above described example and the examples to follow, the measurements are performed on the subject's leg as this maximises the effect of any blood pooling, thereby maximising the effectiveness of the measurement procedure to determine indicators that can be used in identifying venous insufficiency.

Examples of how the measurements are performed with respect to the orientation of the subject will now be described in further detail.

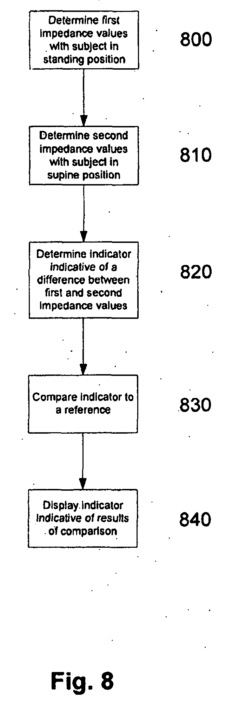

In the specific example of Figure 8, at step 800 first impedance values are determined with the subject in a standing position to maximise blood pooling. The orientation of the subject in this step can include the subject standing, leaning or sitting with their leg hanging in a substantially vertical position. The first impedance measurement is typically performed after the subject has been standing for a predetermined time period, such as five to fifteen minutes, to maximize blood pooling, however this is not essential.

The subject is then reoriented into a supine position, so that the pooled blood is able to redistribute, and at step 810 second impedance values are determined with the subject in the supine position. The orientation in this step can be any other orientation designed to reduce or minimise blood pooling, such as elevation of the legs from a sitting position, or elevation of the legs to a height of up to 20 cm above the level of their heart whilst in a supine position. For the purpose of the remaining description, the term supine will be understood to encompass any position that minimises pooling of blood in the subject's leg. The second impedance measurements can be performed immediately after the orientation change, as the redistribution of body fluids commences rapidly thereafter. The second impedance measurements can also be performed a predetermined time after the orientation change.

The value of the resistance of intracellular fluid is determined for each of the first and second impedance measurements as described with reference to Figure 7 above and at step 820 the processing system 102 determines an indicator based on the changes in the intracellular fluid levels resulting from of the orientation change.

At step 830, the indicator is compared to a reference, which can be based on similar indicator values derived from sample populations, or the like. Alternatively, the reference can be based on first and second impedance values previously determined for the subject, for example prior to the onset of venous insufficiency, allowing longitudinal analysis to be performed.

At step 840, the results of the comparison are displayed to the subject for use in the assessment of venous insufficiency and/or oedema.

It will be appreciated that the indicator in this specific example is indicative of a change in intracellular fluid levels between a maximum pooling baseline measurement and a measurement after an orientation change. This allows straightforward comparison to reference values from measurements performed for a population of subjects.

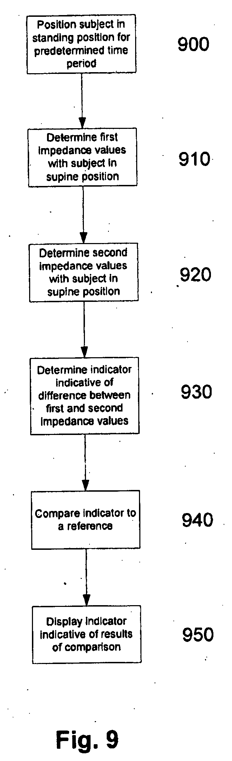

In the specific example of Figure 9, at step 900 the subject is positioned in a standing position for a predetermined time period, such as five to fifteen minutes, to maximize blood pooling. However, it is not essential to precisely control the amount of time that the subject stands for if the subject has previously been in an upright position. For example, if the subject has been waiting in a standing or sitting position before the measurements commence it is likely that blood pooling has already taken place and in this case standing for a further period of time would have little effect on the degree of blood pooling. On the other hand, if the subject has been in a supine position or the subject's legs have been raised prior to the measurements, standing for a predetermined period of time will be necessary to maximise the blood pooling before the measurements.

With blood pooling in the subject maximized, the orientation of the subject is changed to a supine position, and first impedance values are determined at step 910 at a first time. Second impedance values are then determined at step 920 at a second time. Again, the first and second impedance values are indicative of intracellular fluid levels, and therefore could be based on a plurality of impedance measurements at a plurality of frequencies, or the impedance parameter values $\alpha$, $R_0$ and $R_\infty$, as derived from impedance measurements in some manner.

It will be appreciated that in this example the first and second impedance values are determined while the subject is in a single orientation, with the changing body fluid levels being induced by the orientation change prior to the measurements being performed.

At step 930, an indicator is determined based on first and second impedance values. Again, this indicator will typically be indicative of the change in the subject's body fluid levels between the measurements, and could be based on the indicators $I$ outlined above. It will be appreciated that the change in the body fluids levels can be used to determine average rate of change in the body fluid levels over time, since the measurements are performed with the subject in a single orientation in this example.

Again, the indicator is compared to a reference at step 940, and the results of the comparison are displayed to the subject for use in the assessment of venous insufficiency and/or oedema at step 950.

Results of the comparison can be displayed to allow the relevance of any change to be assessed. In this regard, if the comparison indicates that the decrease in the fluid levels is larger than an amount determined from the reference, then this indicates that there was significant blood pooling within the subject whilst in the standing position that was able to rapidly redistribute after the orientation change, which is in turn indicative of venous insufficiency. It will be appreciated from this, that the magnitude of the difference between the first and second impedance values can be indicative of the degree of venous insufficiency.

Whilst in the above example the subject is initially standing, with measurements being made in the supine position, this is not essential, and alternatively, the subject could be provided in the supine position to minimise blood pooling prior to a measurement being performed. Following this, the subject is positioned to maximise blood pooling, such by having the subject stand, so that the first and second measurements reflect the rate of blood pooling in the leg.

Optionally, additional impedance values can be taken so that a plurality of impedance values is determined in a single orientation. For example, a sequence of impedance measurements may be determined with a predetermined period of time between each measurement, such as 30 seconds, and impedance values determined for each impedance measurement in the sequence.

A series of indicators can then be determined for successive pairs of impedance values, allowing the changes in the subject's body fluid over time to be examined in more detail. Alternatively, an indicator can be determined for the plurality or sequence of impedance values, the indicator being indicative of the rate of change of the body fluid levels over time. In any event, the changes over time, or the rate of change can be used in the assessment of venous insufficiency.

It will be appreciated that the use of more than two impedance values allows a more detailed profile of the changes in the body fluid levels to be examined. For example, a plot of the impedance values over time can be generated to illustrate the changes in body fluid levels graphically as a curve. Characteristics of the curve representing the changing body fluid levels can be used to differentiate normal subjects from subjects with venous insufficiency, since the change in body fluid levels will typically be more pronounced immediately after orientation changes in venous insufficiency subject, leading to a curve with a logarithmic shape, where normal subjects will tend to display constant changes over time, leading to a curve with a linear shape.

Rates of change in the body fluid levels may be determined by the processing system to enable more detailed assessment based on the shape of the curve. The indicator is based on a rate of change of intracellular resistance given by:

$$I = \frac{dR_i}{dt} \qquad (8)$$

Similarly, not according to the invention, the indicator could be based on a rate of change of the ratio of intracellular resistance and extracellular resistance is given by:

$$I = \frac{d(R_i/R_e)}{dt} \qquad (9)$$

Determination of the rates of change as derivative functions as discussed above allows direct comparisons of the rates of change to reference values or thresholds. For example, a rate of change value that exceeds a threshold value shortly after an orientation change may be indicative of venous insufficiency.

In another example, a plot of the impedance values over time taken from a set of impedance measurements can be displayed to a user, such as a medical practitioner, to enable a visual assessment to be performed for use in the assessment of the subject's condition. For example, the user may compare the characteristics of the plot with reference values or plots representative of a normal population, another plot of impedance values from the same subject at an earlier point in time. Alternatively, the plot may be displayed superimposed with threshold curves such that venous insufficiency may be present if the plot crosses a threshold. It will be appreciated that the use of plots allows a user to make a more detailed assessment of the subject's condition.

Alternatively, the assessment of a sequence of impedance values can be performed by the processing system to derive indicator values from the sequence of impedance values which are indicative of the changes in the body fluid levels. These indicator values can instead be displayed to the user for further assessment, or compared to reference values to allow a more rapid assessment of the subject's condition.

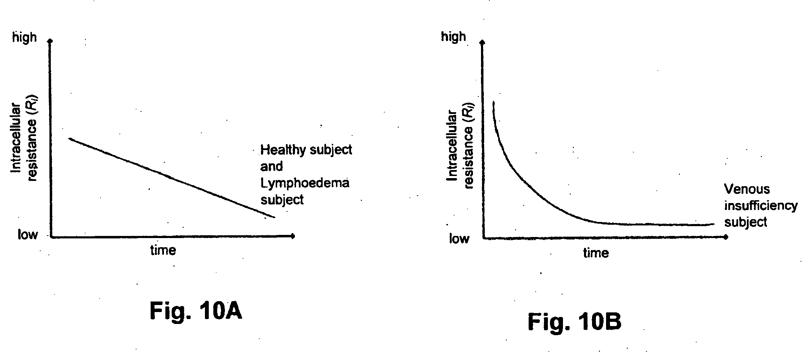

Examples of the particular physiological mechanisms which allow the use of an indicator indicative of changes in body fluid levels in the subject in the assessment of venous insufficiency will now be described with reference to Figures 10A to 10D.

As discussed above, the resistance of the intracellular body fluid, $R_i$ (also referred to as intracellular resistance), can be used as a basis for an indicator for differentiating between subjects with venous insufficiency and normal subjects, or subjects with lymphoedema.

The intracellular resistance has been found to decrease following a change in orientation from a position that maximizes pooling of blood, such as a standing position or a sitting position with dangling lower .legs, to a position that de-loads the legs, such as a supine position or sitting position with one or both lower legs raised horizontally. This decrease in intracellular resistance is a result of the outflow of pooled blood from the lower legs as the pooling effect of gravity is minimized, and due to changes in blood cell orientation during this process.

The magnitude and rate of the decrease in intracellular resistance follows a profile which can be indicative of the condition of the subject. For example, in subjects without venous insufficiency, the decrease in intracellular resistance occurs at a relatively constant rate, but on the other hand, in subjects with venous insufficiency, an initial period of rapid decrease occurs as the blood which has pooled as a result of the malfunctioning valves in the veins of the subject is discharged from the lower limb. Accordingly, a plot of the intracellular resistance in a normal subject will have a linear profile with a relatively constant rate of change as shown in Figure 10A, whilst a similar plot for a venous insufficiency subject will have a pronounced initial decline with a logarithmic profile as shown in Figure 10B.

It will be appreciated that the profile of intracellular resistance following an orientation change can be used to differentiate between normal subjects and subjects with venous insufficiency. Accordingly, an indicator at least partially indicative of the change or rate of change of intracellular resistance can be useful in the assessment of venous insufficiency.

Changes in the resistance of the extracellular body fluid, $R_e$ (also referred to as extracellular resistance) following a change in orientation can additionally be used to assess venous insufficiency. Since extracellular resistance is effectively determined or estimated in the process for determining intracellular resistance, the use of this parameter will not unduly increase the processing burden if intracellular resistance is being used.

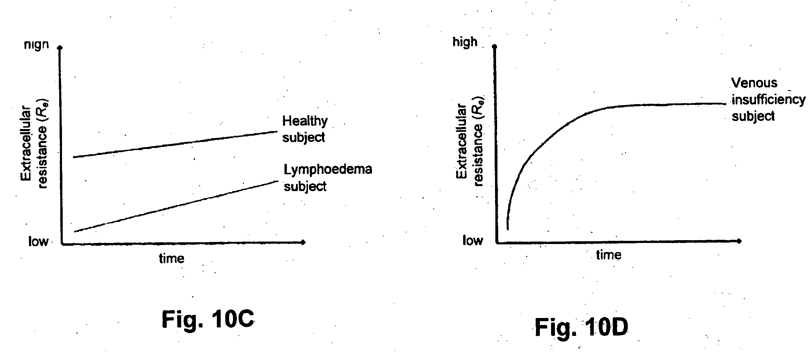

The additional use of extracellular resistance can allow differentiation between normal subjects, subjects with lymphoedema and subjects with venous insufficiency, due to different characteristics in the magnitudes and rates of changes in extracellular resistance for the respective subjects. Examples of plots of extracellular resistance for normal subjects and subjects with lymphoedema are shown in Figure 10C, and a plot for a subject with venous insufficiency is shown in Figure 10D.

In one example, not according to the invention, the indicator is indicative of the respective changes in intracellular and extracellular fluid levels in the subject, such that the differences in characteristics of intracellular and extracellular resistances can be used to distinguish conditions.

It will also be appreciated that the respective methods described with reference to Figures 8 and 9 above can be combined, such that a baseline measurement of maximum blood pooling is performed followed by a two or more measurements after and orientation change to allow assessment of the body fluid level changes. Impedance measurements can be determined periodically over an expended period of time throughout which the orientation of the subject is changed at least once, so that the assessment of venous insufficiency can be based on a series of impedance values.

It will be appreciated that the impedance measurement techniques described above can also be applied to first and second orientations other than standing and supine if a change between the orientations promotes a redistribution in the levels of body fluids in the subject. For example, any changes in the orientation of one leg between positions which promote draining or pooling of body fluids in the leg can be used in conjunction with the above described methods.

An example method of altering the level of pooling in the legs is to have only the legs change orientation, while the torso of the subject remains in a constant orientation. The torso of the subject may be horizontal, such that the subject is lying down with only the position of one of the legs changing position. On the other hand, the torso of the subject may be vertical, such that the subject is sitting or standing with the only the position of one of the legs changing position.

In one example, the subject is positioned into a first orientation in which the subject is lying down with the subject's legs extending horizontally. The subject is then repositioned into a second orientation in which the subject is lying down with a leg raised at an angle to the horizontal. Raising one of the legs promotes draining of body fluids from the raised leg into the body, and in this example the changes in the resistance of the body fluids as they drain from the raised leg can be used to determine an indicator. The impedance measurements may be performed in the first and/or second orientation using the methods described above.

Optionally, the subject's leg can be supported while raised so that no exertion from the subject is required in order to maintain the correct orientation. This approach allows the patient to remain in a comfortable lying position throughout the duration of the measurements. This is beneficial for patients that may have difficulty in standing for prolonged periods.

In another example, one of the subject's legs can be lowered to an angle below the horizontal to promote pooling in that leg, while the subject remains in a lying position. Alternatively, the subject can be sitting and can be oriented to raise one leg while the other leg is lowered.

It will also be appreciated that similar methods can utilise raising and lowering of both limbs simultaneously. For example, the subject can remain in the lying position on an adjustable bed, to allow one or more sections of the bed can be tilted at an angle to the horizontal and thus promote draining or pooling of body fluids in the leg.

In another example, a first impedance measurement is performed with the subject positioned in a first orientation with the subject lying down with one leg raised, and a second impedance measurement is performed with the subject positioned in a second orientation with the subject still lying down but with the other leg raised. It will be appreciated that the body fluids in the legs of the subject will redistribute between the times of the first and second measurement as a result of the orientation change and therefore an indicator may be determined based on the measurements which is indicative of the change in body fluids when legs are alternatively raised.

For example, a measurement performed with one leg raised can be compared to a measurement performed with the other leg raised to indicate whether one of the legs has undergone more pronounced draining than the other leg. This can be used to help determine whether a subject has venous insufficiency in one leg only.

In the above described examples, the term impedance generally refers to a measured impedance value or impedance parameter value derived therefrom. The term resistance refers to any measured value relating to the impedance, such as admittance of reactance measurements. It will also be appreciated that the term impedance measurement covers admittance and other related measurements.

The term processing system is intended to include any component capable of performing processing and can include any one or more of a processing system and a computer system.

Features from different examples above may be used interchangeably where appropriate. Thus, for example, multiple different indicators may be determined and compared to respective thresholds.

Furthermore, whilst the above examples have focussed on a subject such as a human, it will be appreciated that the measuring device and techniques described above can be used with any animal, including but not limited to, primates, livestock, performance animals, such race horses, or the like.

The above described processes can be used in determining biological indicators, which in turn can be used for diagnosing the presence, absence or degree of a range of conditions and illnesses, including, but not limited to oedema, lymphoedema, body composition, or the like.

Furthermore, whilst the above described examples have focussed on the application of a voltage signal to cause a current to flow through the subject, this is not essential and the process can also be used when applying a current signal.

## Claims

1. A method for use in analysing impedance measurements performed on a subject(s), the subject being arranged such that body fluid levels in at least one leg segment of the subject changes between a first time and a second time, the method including, in a processing system:

   a) at the first time (200), determining at least one first impedance value indicative of the impedance of the at least one leg segment of the subject;
   b) at the second time (210), determining at least one second impedance value indicative of the impedance of the at least one leg segment of the subject; and,
   c) determining an indicator (220) based on the at least one first and at least one second impedance values, the indicator being at least partially indicative of a rate of change of intra-cellular fluid levels in the at least one leg segment, and being usable in the assessment of venous insufficiency; **characterised in that** the method further includes:

   d) comparing the rate of change to a threshold value (830); and
   e) providing an indication of the results of the comparison (840), wherein if the rate of change exceeds the threshold value this is indicative of a presence or degree of venous insufficiency.

2. A method according to claim 1, wherein the indicator is indicative of a change between the at least one first impedance value and the at least one second impedance value.

3. A method according to claim 1 or claim 2, wherein the method includes, in the processing system:

   a) comparing the indicator to a reference; and,
   b) providing an indication of the results of the comparison to allow determination of a presence or degree of venous insufficiency.

4. A method according claim any one of claims 1 to 3, wherein the method includes, in the processing system:

   a) determining the at least one first impedance value with the subject in a first orientation; and
   b) at least one of:

   i) determining the at least one second impedance value with the subject in a second orientation; and,
   ii) after positioning the subject in a second orientation for a predetermined time period, determining the at least one second impedance value with the subject in the first orientation.

5. A method according to any one of claims 1 to 3, wherein the method includes:

   a) positioning the subject in a first orientation for a predetermined time period; and,
   b) positioning the subject in a second orientation;

and wherein the method further includes, in the processing system:

    c) determining the at least one first and second impedance values with the subject in the second orientation.

6.  A method according to claim 4 or claim 5, wherein the torso of the subject remains in a constant orientation, and when the subject is in the first orientation a first leg of the subject is positioned in a first position, and when the subject is in the second orientation the first leg of the subject is positioned in a second position.

7.  A method according to any one of claims 1 to 6, wherein the method includes, in the processing system:

    a) examining at least one change in the impedance values over time;
    b) determining whether a rate of change in impedance values is at least one of:

        i) constant;
        ii) non-constant; and,
        iii) logarithmic.

to thereby allow the at least one change in the impedance values to be used in the assessment of venous insufficiency.

8.  A method according to any one of claims 1 to 7, wherein the method includes, in the processing system:

    a) determining the at least one first impedance value using a plurality of impedance measurements performed at a plurality of different frequencies; and,
    b) determining the at least one second impedance value using a plurality of impedance measurements performed at a plurality of different frequencies.

9.  A method according to any one of claims 1 to 8, wherein at least one impedance measurement is measured at a measurement frequency of at least one of:

    a) less than 100 kHz;
    b) less than 50 kHz; and,
    c) less than 10 kHz.

wherein the method includes, in the processing system, using the at least one impedance measurement as an estimate of a resistance of the subject at a zero measurement frequency.

10.  A method according to any one of the claims 1 to 9, wherein at least one impedance measurement is measured at a measurement frequency of at least one of:

    a) greater than 200 kHz;
    b) greater than 500 kHz; and,
    c) greater than 1000 kHz.

wherein the method includes, in the processing system, using the at least one impedance measurement as an estimate of a resistance of the subject at an infinite measurement frequency.

11.  A method according to any one of the claims 1 to 10, wherein the method includes, in the processing system, determining the indicator using at least one of the equations:

$$I = \frac{\Delta R_i}{\Delta R_e}$$

$$I = \Delta R_i$$

$$I = \frac{d\left(R_i / R_e\right)}{dt}$$

$$I = \frac{dR_i}{dt}$$

where:

$I$ is the indicator $\Delta R_i$ is a change in the resistance of intracellular fluid, and
$\Delta R_e$ is a change in the resistance of intracellular fluid, with $R_e = R_0$.

**12.** A method according to any one of the claims 1 to 11, wherein the method includes, in the processing system, causing the impedance measurements to be performed by:

a) causing one or more electrical signals to be applied to the subject using a first set of electrodes;
b) measuring electrical signals across a second set of electrodes applied to the subject in response to the applied one or more signals; and,
c) determining from the applied signals and the measured signals at least one measured impedance value.

**13.** Apparatus (300) for use in analysing impedance measurements performed on a subject(s), the apparatus including a processing system (310) for:

a) at a first time (200), determining at least one first impedance value indicative of the impedance of the at least one leg segment of the subject;
b) at a second time (210); determining at least one second impedance value indicative of the impedance of the at least one leg segment of the subject; and,
c) determining an indicator (220) based on the at least one first and at least one second impedance values, the indicator being at least partially indicative of a rate of change of intra-cellular fluid levels in the at least one leg segment, and being usable in the assessment of venous insufficiency; the processing system further **characterised by** being configured for:

d) comparing the rate of change to a threshold value (830), and,
e) providing an indication of the results of the comparison (840), wherein if the rate of change exceeds the threshold value, this is indicative of a presence or degree of venous insufficiency.

**14.** Apparatus according to claim 13, wherein the apparatus includes:

a) a signal generator for generating electrical signals;
b) a sensor for sensing electrical signals; and,
c) a processing system for:

i) causing one or more electrical signals to be applied to the subject using a first set of electrodes;
ii) measuring electrical signals across a second set of electrodes applied to the subject in response to the applied one or more signals; and,
iii) determining from the applied signals and the measured signals at least one measured impedance value.

**Patentansprüche**

**1.** Verfahren für die Verwendung bei der Analyse von an einem Subjekt (S) durchgeführten Impedanzmessungen, wobei das Subjekt so angeordnet ist, dass sich Körperfluidspiegel in wenigstens einem Beinsegment des Subjekts zwischen einem ersten Zeitpunkt und einem zweiten Zeitpunkt verändern, wobei das Verfahren in einem Verarbeitungssystem umfasst:

a) zu einem ersten Zeitpunkt (200) Bestimmen wenigstens eines ersten Impedanzwerts, der für die Impedanz des wenigstens einen Beinsegments des Subjekts indikativ ist;

b) zu einem zweiten Zeitpunkt (210) Bestimmen wenigstens eines zweiten Impedanzwerts, der für die Impedanz des wenigstens einen Beinsegments des Subjekts indikativ ist; und

c) Bestimmen eines Indikators (220) auf der Grundlage des wenigstens einen ersten und des wenigstens einen zweiten Impedanzwerts, wobei der Indikator wenigstens zum Teil indikativ für eine Veränderungsrate von intrazellulären Fluidspiegeln in dem wenigstens einen Beinsegments ist und für die Beurteilung von Veneninsuffizienz verwendbar ist; **dadurch gekennzeichnet, dass** das Verfahren ferner umfasst:

d) Vergleichen der Veränderungsrate mit einem Schwellenwert (830); und

e) Bereitstellen einer Anzeige der Ergebnisse des Vergleichs (840), wobei dies, wenn die Veränderungsrate den Schwellenwert übersteigt, indikativ für ein Vorhandensein oder einen Grad von Veneninsuffizienz ist.

2. Verfahren gemäß Anspruch 1, wobei der Indikator für eine Veränderung zwischen dem wenigstens einen ersten Impedanzwert und dem wenigstens einen zweiten Impedanzwert indikativ ist.

3. Verfahren gemäß Anspruch 1 oder Anspruch 2, wobei das Verfahren in dem Verarbeitungssystem umfasst:

a) Vergleichen des Indikators mit einer Referenz; und

b) Bereitstellen einer Anzeige der Ergebnisse des Vergleichs, um das Bestimmen eines Vorhandenseins oder Grads von Veneninsuffizient zu ermöglichen.

4. Verfahren gemäß einem der Ansprüche 1 bis 3, wobei das Verfahren in dem Verarbeitungssystem umfasst:

a) Bestimmen des wenigstens einen ersten Impedanzwerts mit dem Subjekt in einer ersten Orientierung; und

b) wenigstens eines von:

i) Bestimmen des wenigstens einen zweiten Impedanzwerts mit dem Subjekt in einer zweiten Orientierung; und

ii) nach dem Positionieren des Subjekts in einer zweiten Orientierung für einen vorbestimmten Zeitraum Bestimmen des wenigstens einen zweiten Impedanzwerts mit dem Subjekt in der ersten Orientierung.

5. Verfahren gemäß einem der Ansprüche 1 bis 3, wobei das Verfahren umfasst:

a) Positionieren des Subjekts in einer ersten Orientierung für einen vorbestimmten Zeitraum; und

b) Positionieren des Subjekts in einer zweiten Orientierung;

und wobei das Verfahren ferner in dem Verarbeitungssystem umfasst:

c) Bestimmen der wenigstens einen ersten und zweiten Impedanzwerte mit dem Subjekt in der zweiten Orientierung.

6. Verfahren gemäß Anspruch 4 oder Anspruch 5, wobei der Torso des Subjekts in einer konstanten Orientierung bleibt, und wenn sich das Subjekt in der ersten Orientierung befindet, ein erstes Bein des Subjekts in einer ersten Position positioniert ist, und wenn sich das Subjekt in der zweiten Orientierung befindet, das erste Bein des Subjekts in einer zweiten Position positioniert ist.

7. Verfahren gemäß einem der Ansprüche 1 bis 6, wobei das Verfahren in dem Verarbeitungssystem umfasst:

a) Untersuchen von wenigstens einer Veränderung der Impedanzwerte im Zeitverlauf;

b) Bestimmen, ob eine Veränderungsrate von Impedanzwerten wenigstens eines von:

i) konstant;

ii) nichtkonstant; und

iii) logarithmisch

ist, um damit zu ermöglichen, dass die wenigstens eine Veränderung der Impedanzwerte bei der Beurteilung von Veneninsuffizienz verwendet wird.

8. Verfahren gemäß einem der Ansprüche 1 bis 7, wobei das Verfahren in dem Verarbeitungssystem umfasst:

   a) Bestimmen des wenigstens einen ersten Impedanzwerts unter Verwendung einer Vielzahl von Impedanzmessungen, die mit einer Vielzahl von verschiedenen Frequenzen durchgeführt werden; und
   b) Bestimmen des wenigstens einen zweiten Impedanzwerts unter Verwendung einer Vielzahl von Impedanzmessungen, die mit einer Vielzahl von verschiedenen Frequenzen durchgeführt werden.

9. Verfahren gemäß einem der Ansprüche 1 bis 8, wobei wenigstens eine der Impedanzmessungen mit einer Messfrequenz von wenigstens einem von:

   a) weniger als 100 kHz;
   b) weniger als 50 kHz; und
   c) weniger als 10 kHz

gemessen wird, wobei das Verfahren in dem Verarbeitungssystem die Verwendung der wenigstens einer Impedanzmessung als Abschätzung eines Widerstandswerts des Subjekts bei einer Messfrequenz von null umfasst.

10. Verfahren gemäß einem der Ansprüche 1 bis 9, wobei wenigstens eine Impedanzmessung mit einer Messfrequenz von wenigstens einem von:

    a) höher als 200 kHz;
    b) höher als 500 kHz; und
    c) höher als 1000 kHz

gemessen wird, wobei das Verfahren in dem Verarbeitungssystem die Verwendung der wenigstens einer Impedanzmessung als Abschätzung eines Widerstandswerts des Subjekts bei einer Messfrequenz von unendlich umfasst.

11. Verfahren gemäß einem der Ansprüche 1 bis 10, wobei das Verfahren in dem Verarbeitungssystem das Bestimmen des Indikators unter Verwendung wenigstens einer der Gleichungen:

$$I = \frac{\Delta R_i}{\Delta R_e}$$

$$I = \Delta R_i$$

$$I = \frac{d\left(R_i / R_e\right)}{dt}$$

$$I = \frac{dR_i}{dt}$$

umfasst, wobei:

   $I$ der Indikator ist,
   $\Delta R_i$ eine Veränderung des Widerstandswerts von intrazellulärem Fluid ist und
   $\Delta R_e$ eine Veränderung des Widerstandswerts von intrazellulärem Fluid ist, mit $R_e = R_0$.

12. Verfahren gemäß einem der Ansprüche 1 bis 11, wobei das Verfahren in dem Verarbeitungssystem umfasst, zu bewirken, dass die Impedanzmessungen durch:

a) Bewirken, dass unter Verwendung eines ersten Satzes von Elektroden ein oder mehrere elektrische Signale an das Subjekt angelegt werden;
b) Messen von elektrischen Signalen über einen an das Subjekt angelegten zweiten Satz von Elektroden in Antwort auf das eine oder die mehreren angelegten Signale; und
c) aus den angelegten Signalen und den gemessenen Signalen Bestimmen wenigstens eines gemessenen Impedanzwerts

durchgeführt wird.

**13.** Vorrichtung (300) für die Verwendung bei der Analyse von an einem Subjekt (S) durchgeführten Impedanzmessungen, wobei die Vorrichtung ein Verarbeitungssystem (810) umfasst zum:

a) zu einem ersten Zeitpunkt (200) Bestimmen wenigstens eines ersten Impedanzwerts, der für die Impedanz des wenigstens einen Beinsegments des Subjekts indikativ ist;
b) zu einem zweiten Zeitpunkt (210) Bestimmen wenigstens eines zweiten Impedanzwerts, der für die Impedanz des wenigstens einen Beinsegments des Subjekts indikativ ist; und
c) Bestimmen eines Indikators (220) auf der Grundlage des wenigstens einen ersten und des wenigstens einen zweiten Impedanzwerts, wobei der Indikator wenigstens zum Teil indikativ für eine Veränderungsrate von intrazellulären Fluidspiegeln in dem wenigstens einen Beinsegments ist und für die Beurteilung von Veneninsuffizienz verwendbar ist; wobei das Verarbeitungssystem ferner **dadurch gekennzeichnet ist, dass** es gestaltet ist zum:

d) Vergleichen der Veränderungsrate mit einem Schwellenwert (830); und
e) Bereitstellen einer Anzeige der Ergebnisse des Vergleichs (840), wobei dies, wenn die Veränderungsrate den Schwellenwert übersteigt, indikativ für ein Vorhandensein oder einen Grad von Veneninsuffizienz ist.

**14.** Vorrichtung gemäß Anspruch 13, wobei die Vorrichtung umfasst:

a) einen Signalgenerator zum Erzeugen elektrischer Signale;
b) einen Sensor zum Abfühlen elektrischer Signale; und
c) ein Verarbeitungssystem zum:

i) Bewirken, dass unter Verwendung eines ersten Satzes von Elektroden ein oder mehrere elektrische Signale an das Subjekt angelegt werden;
ii) Messen von elektrischen Signalen über einen an das Subjekt angelegten zweiten Satz von Elektroden in Antwort auf das eine oder die mehreren angelegten Signale; und
iii) aus den angelegten Signalen und den gemessenen Signalen Bestimmen wenigstens eines gemessenen Impedanzwerts.

## Revendications

**1.** Procédé destiné à être utilisé dans l'analyse de mesures d'impédance effectuées sur un ou plusieurs sujets, le sujet étant placé de manière à ce que des niveaux de fluide corporel dans au moins un segment de jambe du sujet changent entre un premier temps et un second temps, le procédé comprenant, dans un système de traitement, les étapes suivantes :

a) au premier temps (200), déterminer au moins une première valeur d'impédance indicative de l'impédance de l'au moins un segment de jambe du sujet ;
b) au second temps (210), déterminer au moins une seconde valeur d'impédance indicative de l'impédance de l'au moins un segment de jambe du sujet ; et
c) déterminer un indicateur (220) basé sur les au moins une première et une seconde valeurs d'impédance, l'indicateur étant au moins partiellement indicatif d'une vitesse de changement de niveaux de fluides intracellulaires dans l'au moins un segment de jambe, et étant utilisable dans l'évaluation d'une insuffisance veineuse ; **caractérisé en ce que** le procédé comprend en outre les étapes suivantes :

d) comparer la vitesse de changement d'une valeur de seuil (830) ; et
e) fournir une indication des résultats de la comparaison (840), où si la vitesse de changement dépasse la valeur de seuil, cela est indicatif de la présence ou d'un certain degré d'insuffisance veineuse.

2. Procédé selon la revendication 1, dans lequel l'indicateur est indicatif d'un changement entre l'au moins une première valeur d'impédance et l'au moins une seconde valeur d'impédance.

3. Procédé selon la revendication 1 ou la revendication 2, dans lequel le procédé comprend, dans le système de traitement, les étapes suivantes :

a) comparer l'indicateur à une référence ; et,
b) fournir une indication des résultats de la comparaison pour permettre la détermination d'une présence ou d'un certain degré d'insuffisance veineuse.

4. Procédé selon l'une quelconque des revendications 1 à 3, dans lequel le procédé comprend, dans le système de traitement, les étapes suivantes :

a) déterminer l'au moins une première valeur d'impédance avec le sujet dans une première orientation ; et
b) au moins :

i) déterminer l'au moins une seconde valeur d'impédance avec le sujet dans une seconde orientation ; et
ii) après avoir positionné le sujet dans une seconde orientation pendant une durée prédéterminée, déterminer l'au moins une seconde valeur d'impédance avec le sujet dans une seconde orientation.

5. Procédé selon l'une quelconque des revendications 1 à 3, dans lequel le procédé comprend les étapes suivantes :

a) positionner le sujet dans une première orientation pendant une durée prédéterminée ; et
b) positionner le sujet dans une seconde orientation ; dans lequel le procédé comprend en outre, dans le système de traitement, les étapes suivantes :

c) déterminer les au moins une première et au moins une seconde valeurs d'impédance avec le sujet dans la seconde orientation.

6. Procédé selon la revendication 4 ou la revendication 5, dans lequel le torse du sujet reste avec une orientation constante, et lorsque le sujet est dans la première orientation, une première jambe du sujet est positionnée dans une première position, et lorsque le sujet est dans la seconde orientation, la première jambe du sujet est positionnée dans une seconde position.

7. Procédé selon l'une quelconque des revendications 1 à 6, dans lequel le procédé comprend, dans le système de traitement, les étapes suivantes :

a) examiner au moins un changement dans les valeurs d'impédance au cours du temps ;
b) déterminer si une vitesse de changement des valeurs d'impédance présente au moins une des caractéristiques suivantes :

i) constante ;
ii) non constante ; et
iii) logarithmique,

pour ainsi permettre à l'au moins un changement de valeurs d'impédance d'être utilisé dans l'évaluation d'une insuffisance veineuse.

8. Procédé selon l'une quelconque des revendications 1 à 7, dans lequel le procédé, dans le système de traitement, comprend les étapes suivantes :

a) déterminer l'au moins une première valeur d'impédance en utilisant une pluralité de mesures d'impédance effectuées à une pluralité de fréquences différentes ; et
b) déterminer l'au moins une seconde valeur d'impédance en utilisant une pluralité de mesures d'impédance effectuées à une pluralité de fréquences différentes.

**9.** Procédé selon l'une quelconque des revendications 1 à 8, dans lequel l'au moins une mesure d'impédance est mesurée à une fréquence de mesure satisfaisant au moins une des conditions suivantes :

a) inférieure à 100 kHz ;
b) inférieure à 50 kHz ; et
c) inférieure à 10 kHz,

où le procédé comprend, dans le système de traitement, d'utiliser l'au moins une mesure d'impédance comme une estimation d'une résistance du sujet à une fréquence de mesure nulle.

**10.** Procédé selon l'une quelconque des revendications 1 à 9, dans lequel au moins une mesure d'impédance est mesurée à une fréquence de mesure satisfaisant au moins une des conditions suivantes :

a) supérieure à 200 kHz ;
b) supérieure à 500 kHz ; et
c) supérieure à 1000 kHz,

où le procédé comprend, dans le système de traitement, d'utiliser l'au moins une mesure d'impédance comme une estimation d'une résistance du sujet à une fréquence de mesure infinie.

**11.** Procédé selon l'une quelconque des revendications 1 à 10, dans lequel le procédé comprend, dans le système de traitement, de déterminer l'indicateur en utilisant au moins une des équations suivantes :

$$I = \frac{\Delta R_i}{\Delta R_e}$$

$$I = \Delta R_i$$

$$I = \frac{d(R_i/R_e)}{dt}$$

$$I = \frac{dR_i}{dt}$$

où :

$I$ est l'indicateur
$\Delta R_i$ est un changement de la résistance du fluide intracellulaire, et
$\Delta R_e$ est un changement de la résistance du fluide intracellulaire, avec $R_e = R_0$.

**12.** Procédé selon l'une quelconque des revendications 1 à 11, dans lequel le procédé comprend, dans le système de traitement, d'amener les mesures d'impédance à être effectuées en :

a) amenant un ou plusieurs signaux électriques à être appliqués au sujet au moyen d'un premier jeu d'électrodes ;
b) mesurant les signaux électriques à travers un second jeu d'électrodes appliqués au sujet en réponse à l'un ou aux signaux appliqués ; et
c) déterminant, à partir des signaux appliqués et des signaux mesurés, au moins une valeur d'impédance mesurée.

**13.** Appareil (300) destiné à être utilisé dans l'analyse de mesures d'impédance effectuées sur un ou plusieurs sujets, l'appareil comprenant un système de traitement (310) pour :

a) au premier temps (200), déterminer au moins une première valeur d'impédance indicative de l'impédance de l'au moins un segment de jambe du sujet ;

b) au second temps (210), déterminer au moins une seconde valeur d'impédance indicative de l'impédance de l'au moins un segment de jambe du sujet ; et

c) déterminer un indicateur (220) basé sur les au moins une première et une seconde valeurs d'impédance, l'indicateur étant au moins partiellement indicatif d'une vitesse de changement de niveaux de fluides intracellulaires dans l'au moins un segment de jambe, et étant utilisable dans l'évaluation d'une insuffisance veineuse ; le système de traitement étant en outre **caractérisé en ce qu'**il est configuré pour :

d) comparer la vitesse de changement d'une valeur de seuil (830) ; et

e) fournir une indication des résultats de la comparaison (840), où si la vitesse de changement dépasse la valeur de seuil, cela est indicatif de la présence ou d'un certain degré d'insuffisance veineuse.

**14.** Appareil selon la revendication 13, dans lequel l'appareil comprend :

a) un générateur de signal pour générer des signaux électriques ;

b) un détecteur pour détecter des signaux électriques ;

c) un système de traitement pour :

i) amener un ou plusieurs signaux électriques à être appliqués au sujet au moyen d'un premier jeu d'électrodes ;

ii) mesurer les signaux électriques à travers un second jeu d'électrodes appliqués au sujet en réponse à l'un ou aux signaux appliqués ; et

iii) déterminer, à partir des signaux appliqués et des signaux mesurés, au moins une valeur d'impédance mesurée.

**Fig. 1**

Fig. 2

Fig. 3

**Fig. 4**

Place electrodes
on the subject                    500

Select impedance
measurement type                  510
using computer
system 310

The processing
system 330 selects               520
next measurement
frequency $f_i$

Apply first signal               530
to the subject S

Measure current
through and                       540
voltage induced
across subject S

No          All frequencies       550
            complete

                Yes

Determine
Impedance                         560
measurement
value(s)

Optionally determine
one or more                       570
Impedance
parameter values

# Fig. 5

31

**Fig. 6A**

**Fig. 6B**

Determine first
measured
impedance values at
a first time

700

Determine first
impedance
parameter values
$R_0$ and $R_\infty$

710

Determine first
value of resistance
of intracellular fluid

720

Determine second
measured
impedance values at
a second time

730

Determine second
impedance
parameter values
$R_0$ and $R_\infty$

740

Determine second
value of resistance
of intracellular fluid

750

Determine indicator
based on the first and
second values of
resistance of
intracellular fluid

760

Display indicator to
allow assessment of
venous insufficiency

770

**Fig. 7**

| | |
|---|---|
| Determine first Impedance values with subject in standing position | 800 |
| Determine second impedance values with subject in supine position | 810 |
| Determine indicator indicative of a difference between first and second impedance values | 820 |
| Compare indicator to a reference | 830 |
| Display indicator indicative of results of comparison | 840 |

**Fig. 8**

Position subject in
standing position for    900
predetermined time
period

Determine first
impedance values    910
with subject in
supine position

Determine second
impedance values    920
with subject in
supine position

Determine indicator
indicative of
difference between    930
first and second
impedance values

Compare indicator to    940
a reference

Display indicator
indicative of results    950
of comparison

# Fig. 9

Fig. 10B

Fig. 10A

**Fig. 10D**

Extracellular resistance ($R_e$)

high — low

Venous insufficiency subject

time

**Fig. 10C**

Extracellular resistance ($R_e$)

high — low

Healthy subject

Lymphoedema subject

time

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060111652 A
- US 20050177062 A
- WO 0079255 A
- AU 09000163 W

**Non-patent literature cited in the description**

- **NICOLAIDES AN.** Investigation of Chronic Venous Insufficiency: A Consensus Statement. *Circulation,* 2000, vol. 102, 126-163
- **H. SCHARFETTER et al.** Effect of postural changes on the reliability of volume estimations from bioimpedance spectroscopy data. *Kidney International,* 1997, vol. 51, 1078-1087